(19) Europäisches Patentamt
European Patent Office
Office européen des brevets

(11) Veröffentlichungsnummer: **0 231 901 B1**

(12) **EUROPÄISCHE PATENTSCHRIFT**

(45) Veröffentlichungstag der Patentschrift: **09.10.91**

(51) Int. Cl.5: **C07C 231/00**, C07C 233/15

(21) Anmeldenummer: **87101294.4**

(22) Anmeldetag: **30.01.87**

# (54) Verfahren zur Reinigung von N-Vinylformamid und daraus hergestellte Poly-N-vinylformamide.

(30) Priorität: **05.02.86 DE 3603450**

(43) Veröffentlichungstag der Anmeldung:
**12.08.87 Patentblatt 87/33**

(45) Bekanntmachung des Hinweises auf die Patenterteilung:
**09.10.91 Patentblatt 91/41**

(84) Benannte Vertragsstaaten:
**AT BE CH DE ES FR GB GR IT LI LU NL SE**

(56) Entgegenhaltungen:
**EP-A- 0 184 074**

(73) Patentinhaber: **BASF Aktiengesellschaft**
**Carl-Bosch-Strasse 38**
**W-6700 Ludwigshafen(DE)**

(72) Erfinder: **Kroener, Michael, Dr.**
**Eislebener Weg 8**
**W-6800 Mannheim 31(DE)**
Erfinder: **Schmidt, Willi**
**Uhlandstrasse 74 a**
**W-6700 Ludwigshafen(DE)**
Erfinder: **Oftring, Alfred, Dr.**
**Berner Weg 26**
**W-6700 Ludwigshafen(DE)**
Erfinder: **Proll, Theo, Dr.**
**Mozartstrasse 30**
**W-6702 Bad Duerkheim(DE)**
Erfinder: **Hartmann, Heinrich, Dr.**
**Weinheimer Strasse 46**
**W-6703 Limburgerhof(DE)**

EP 0 231 901 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

N-Vinylformamid kann beispielsweise nach dem Verfahren der DE-PS 12 24 304 durch Abspaltung von Cyanwasserstoff aus Formylalaninnitril der Formel I

$$\begin{array}{l} CH_3\text{-}CH\text{-}NH\text{-}CHO \qquad (I) \\ \quad\;\; | \\ \quad\;\; CN \end{array}$$

in Gegenwart von Feststoffen als Katalysator unter vermindertem Druck bei Temperaturen von vorzugsweise 450 bis 650 °C hergestellt werden. Gemäß der DE-OS 23 36 977 erhält man N-Vinylformamid auch durch Abspaltung von Methanol aus N-alpha-Methoxyethyl-formamid der Formel

$$\begin{array}{l} CH_3\text{-}CH\text{-}NH\text{-}CHO \qquad (II) . \\ \quad\;\; | \\ \quad\;\; OCH_3 \end{array}$$

Das jeweils durch Pyrolyse erhaltene N-Vinylformamid wird unter vermindertem Druck destilliert. Gemäß Beispiel 9 der DE-OS 23 36 977 erfolgt die Destillation des N-Vinylformamids bei 0,13 mbar und einem Siedepunkt von 41 °C. Für eine Destillation in technischem Maßstab kommen derart niedrige Drücke aus wirtschaftlichen Überlegungen kaum in Betracht. Da N-Vinylformamid thermisch sehr empfindlich ist und leicht polymerisiert, erhält man bei der Anwendung von höheren Drücken bei der Destillation von rohem N-Vinylformamid schlechtere Ausbeuten. Bei höheren Drücken benötigt man nämlich bei der Destillation höhere Sumpftemperaturen, die nicht nur bei Batch-Destillationen, sondern auch bei kontinuierlichen Destillationen zu erheblichen Verlusten des Monomeren durch Polymerisation führen. Die Polymerisationsneigung des N-Vinylformamids läßt sich während der Destillation auch durch Zugabe von Stabilisatoren, vor allem bei höheren Temperaturen, nicht ausreichend unterdrücken.

Aus N-Vinylformamid werden Polymerisate hergestellt, die beispielsweise bei der Papierherstellung bzw. der Flockung von Schlämmen gegenüber bekannten Hilfsmitteln überlegene Eigenschaften aufweisen. Dies trifft insbesondere für die hochmolekularen Polymerisate zu, die jedoch nur dann erhalten werden können, wenn man von besonders reinem N-Vinylformamid ausgehen kann. Bereits geringste Verunreinigungen im N-Vinylformamid, die analytisch nur schwer identifiziert werden können, ergeben Monomerqualitäten, aus denen sich keine Polymerisate mit besonders hohen Molekulargewichten, z.B. charakterisiert durch den K-Wert nach Fikentscher von über 160, herstellen lassen. So erhält man bei der Destillation von rohem N-Vinylformamid unter einem Druck von 0,13 mbar nur unzureichende Monomerqualitäten. Andererseits hat sich gezeigt, daß reines N-Vinylformamid während einer längeren Destillationsperiode zur sog. Popcornpolymerisation neigt. Derartige Popcornpolymerisate stellen vernetzte und daher unlösliche Polymerisate dar, die die Kolonne verstopfen.

Der vorliegenden Erfindung liegt die Aufgabe zugrunde, eine Methode zur Reinigung von N-Vinylformamid zur Verfügung zu stellen, nach der man N-Vinylformamid in einer Qualität erhält, die die Herstellung von Polymerisaten mit besonders hohen Molekulargewichten gestattet, die z.B. mit Hilfe der K-Werte nach Fikentscher charakterisiert werden und die mehr als 160 betragen. Während der Destillation soll die Polymerisation vermieden werden bzw. nicht störend in Erscheinung treten.

Die Aufgabe wird erfindungsgemäß mit einem Verfahren zur Reinigung von N-Vinylformamid durch fraktionierte Destillation des N-Vinylformamids in einer Kolonne unter vermindetem Druck dadurch gelöst, daß man die Destillation in Gegenwart von Formamid unter einem Druck von 0,5 bis 30 mbar, gemessen am Kopf der Kolonne, durchführt und die Destillation so steuert, daß als Destillat N-Vinylformamid mit einem Gehalt von 0,1 bis 15 Gew.-% Formamid anfällt. Die Destillation wird vorzugsweise so gesteuert, daß 1 bis 6 Gew.-% Formamid im Destillat verbleiben. Überraschenderweise stören diese Formamidkonzentrationen die nachfolgende Polymerisation des N-Vinylformamids zu Polymerisaten mit besonders hohen K-Werten nicht, wobei Homopolymerisate von N-Vinylformamid mit einem K-Wert von oberhalb 200 (bestimmt nach Fikentscher in 5 %iger wäßriger Kochsalzlösung bei 25 °C und einer Polymerkonzentration von 0,1 Gew.-%) erhältlich sind.

Nach dem erfindungsgemäßen Verfahren wird in erster Linie rohes N-Vinylformamid gereinigt, das beispielsweise bei den eingangs beschriebenen Herstellverfahren für N-Vinylformamid anfällt. Es enthält neben den jeweils bei der Pyrolyse eingesetzten Ausgangsstoffen - Verbindungen der Formel I bzw. II - mehr oder weniger große Mengen an Spaltprodukten der Pyrolyse. Nach dem erfindungsgemäßen Verfahren kann außerdem ein bereits in anderer Weise destilliertes N-Vinylformamid gereinigt werden, um ein Monomer zu gewinnen, aus dem Polymerisate mit besonders hohen Molekulargewichten hergestellt werden können.

Die Reinigung des rohen N-Vinylformamids erfolgt durch fraktionierte Destillation in einer Kolonne unter vermindertem Druck. Wegen der thermischen

Empfindlichkeit des N-Vinylformamids muß die fraktionierte Destillation unter vermindertem Druck vorgenommen werden. Sie erfolgt in dem Bereich von 0,5 bis 30, vorzugsweise 1 bis 15 mbar, wobei sich diese Angaben auf den Druck beziehen, der am Kopf der Kolonne herrscht. Ein wesentliches Merkmal der vorliegenden Erfindung ist, daß die fraktionierte Destillation, bezogen auf das dabei eingesetzte N-Vinylformamid, so durchgeführt wird, daß das als Destillat am Kopf der Kolonne abgezogene N-Vinylformamid 0,1 bis 15 Gew.-% Formamid enthält. Das rohe N-Vinylformamid, das bei der Destillation eingesetzt wird, enthält 1 bis 70 Gew.-% Formamid. Formamid ist gegenüber N-Vinylformamid inert und stört in den angegebenen Konzentrationen im gereinigten N-Vinylformamid die Polymerisation nicht. Formamid wird vorzugsweise in einer Menge von 5 bis 50 Gew.-%, bezogen auf N-Vinylformamid, bei der Destillation eingesetzt. In Abhängigkeit vom Rohprodukt und den Anforderungen an die Reinheit des N-Vinylformamids beträgt die Zahl der theoretischen Böden der jeweils verwendeten Kolonnen 5 bis 40, vorzugsweise 10 bis 20. Die Destillation kann diskontinuierlich durchgeführt werden, indem man eine Lösung von N-Vinylformamid in Formamid in einem Kolben vorlegt und das N-Vinylformamid zusammen mit den oben angegebenen geringen Mengen an Formamid am Kopf einer Kolonne abnimmt. Vorzugsweise wird die Reinigung jedoch kontinuierlich unter Verwendung von Formamid in der Weise durchgeführt, daß man eine Mischung aus dem zu reinigenden N-Vinylformamid und Formamid im unteren Drittel bis etwa zur Mitte einer Kolonne zuführt und am Kopf der Kolonne N-Vinylformamid zusammen mit vorzugsweise bis zu 6 Gew.-% Formamid abtrennt. Bei dieser Arbeitsweise hat man - wie bei Destillationen üblich - am Fuß der Kolonne einen Kreislauf für das Sumpfprodukt. In diesem Kreislauf sind als wesentliche Bauelemente eine Umwälzpumpe und ein Verdampfer, vorzugsweise ein Fallfilmverdampfer, eingebaut. Aus dem Sumpf wird das bei der Destillation mitverwendete Formamid zusammen mit hochsiedenden Verunreinigungen und gegebenenfalls geringen Mengen an N-Vinylformamid abgezogen. Das aus dem Sumpf abgetrennte Formamid kann erforderlichenfalls aufdestilliert und wieder verwendet werden. Aufgrund des Einsatzes von Formamid wird überraschenderweise eine Polymerisation des N-Vinylformamids während der Destillation ganz bzw. weitgehend vermieden. Aus den als Destillat anfallenden Mischungen (N-Vinylformamid mit einem Gehalt von 0,1 bis 15 Gew.-% Formamid) kann man durch Polymerisieren in wäßrigem Medium bei Temperaturen von vorzugsweise 30 bis 100 °C in Gegenwart von Polymerisationsinitiatoren besonders hochmolekulare Poly-N-Vinylformamide herstellen, deren K-Wert oberhalb von 200 liegt, z.B. in dem Bereich von 205 bis 270. Als Polymerisationsverfahren in wäßrigem Medium kommen die Lösungspolymerisation, die Wasser-in-Öl-Polymerisation sowie die umgekehrte Suspensionspolymerisation in Betracht. Vorzugsweise polymerisiert man N-Vinylformamid, das 1 bis 6 Gew.-% Formamid enthält nach dem Wasser-in-Öl-Polymerisationsverfahren. Je höher der K-Wert der Homopolymerisate ist, desto wirksamer sind die daraus durch Abspaltung der Formylgruppe herstellbaren Produkte, z.B. bei der Anwendung als Flockungsmittel für Schlämme.

Die in den Beispielen angegebenen Teile sind Gewichtsteile, die Angaben in Prozent beziehen sich auf das Gewicht der Stoffe. Die K-Werte der Polymerisate wurden nach Fikentscher, Cellulose-Chemie, Band 13, 48-64 und 71-74 (1932) in 5 %iger wäßriger Kochsalzlösung bei 25 °C und einer Polymerkonzentration von 0,1 Gew.-% bestimmt.

Beispiel 1

In einer kontinuierlich arbeitenden Destillationsanlage, die aus einer 4 m langen Kolonne vom Durchmesser 150 mm besteht und mit Füllkörpern Sulzer BX gefüllt ist, werden im unteren Drittel stündlich 8,0 kg einer Mischung zugeführt, die aus 60 % N-Vinylformamid, 3 % höher als N-Vinylformamid siedenden Bestandteilen und 37 % Formamid besteht. Am Kopf der Kolonne wird ein Druck von 10 mbar aufrecht erhalten und stündlich 2 g p-Phenylendiamin als Stabilisator, gelöst in N-Vinylformamid zudosiert.

Mit Hilfe einer am Fuß der Kolonne befindlichen Umwälzpumpe werden stündlich 400 l des Sumpfprodukts über einen auf 140 °C beheizten Fallfilmverdampfer bei 19 mbar umgepumpt. Im stationären Betrieb stellt sich im Sumpfbereich eine Verweilzeit von 1,0 Stunden und eine Temperatur von 107 °C ein. Als Sumpfprodukt werden stündlich 3,1 kg 92,3 %iges Formamid abgezogen, mit einem Hochsiederanteil von 7,7 %. Am Kopf der Kolonne werden bei einem Rücklaufverhältnis von 3 : 1 stündlich 4,9 kg N-Vinylformamid mit einem Gehalt von 2 % Formamid abdestilliert.

Bei dieser Arbeitsweise geht praktisch kein N-Vinylformamid durch Polymerisation verloren. Das Formamid des Sumpfablaufs kann, ggf. nach Abtrennen der hochsiedenden Bestandteile, zurückgeführt werden. Aus dem so gewonnenen N-Vinylformamid lassen sich durch Polymerisation nach Art einer Wasser-in-Öl-Emulsionspolymerisation (vgl. EP-PS 71050) Polymerisate mit einem K-Wert von 210 herstellen.

Beispiel 2

Der in Beispiel 1 angegebenen Kolonne werden pro Stunde 8,0 kg einer Mischung von 60 % N-Vinylformamid, 35,2 % Formamid, 1,8 % der Verbindung der Formel II und 3,0 % Hochsieder zugeführt. Bei sonst gleichen Bedingungen, wie in Beispiel 1 beschrieben, werden als Sumpfprodukt bei 107 °C stündlich 3,1 kg einer Mischung von 87,6 % Formamid, 4,6 % der Verbindung der Formel II und 7,7 % Hochsieder abgezogen. Am Kopf der Kolonne werden 4,9 kg eines Destillats erhalten, das aus 98 % N-Vinylformamid und 2 % Formamid besteht. Der erzielte Reinheitsgrad des N-Vinylformamids entspricht der Monomerenqualität des Beispiels 1.

Beispiel 3

In einer kontinuierlich arbeitenden Destillationsanlage, die aus einer 4 m langen Kolonne vom Durchmesser 300 mm besteht und mit Füllkörpern Sulzer BX gefüllt ist, werden im unteren Drittel stündlich 20,0 kg einer Mischung zugeführt, die aus 61 % N-Vinylformamid, 32 % Formamid, 4,3 % der Verbindung der Formel I und 2,7 % hochsiedenden Produkten unbekannter Zusammensetzung (solche Mischungen fallen bei der Pyrolyse von Verbindungen der Formel I an) besteht. Am Kopf der Kolonne wird ein Druck von 3 mbar aufrechterhalten und stündlich 2 g p-Phenylendiamin als Stabilisator, gelöst in N-Vinylformamid, zudosiert. Mit Hilfe einer am Fuß der Kolonne befindlichen Umwälzpumpe werden stündlich 800 l des Sumpfprodukts über einen auf 140 °C beheizten Fallfilmverdampfer bei 13 mbar umgepumpt. Als Sumpfprodukt werden im stationären Zustand bei 103 °C stündlich 7,6 kg einer Mischung von 81,6 % Formamid, 11,3 % der Verbindung der Formel I und 7,1 % Hochsieder abgezogen. Am Kopf der Kolonne werden bei einem Rücklaufverhältnis von 3 : 1 stündlich 12,4 kg eines Destillates erhalten, das aus 98,4 % N-Vinylformamid und 1,6 % Formamid besteht.

Aus dem so gewonnenen N-Vinylformamid lassen sich durch Polymerisation nach Art einer Wasser-in-Öl-Emulsionspolymerisation (vgl. EP-PS 71050) Homopolymerisate mit hohem K-Wert herstellen. Wenn man z.B. in einem 2-Liter-4-Halskolben, der mit Rührer, Thermometer, Stickstoff-Einleitungsrohr und Kühler versehen ist, 402,3 g destilliertes Wasser, 4,99 g Kaliumdihydrogenphosphat und 0,164 g Natriumhydroxid vorlegt und hierzu unter Rühren eine Mischung aus 339,98 g eines Kohlenwasserstoffgemisches (Mischung aus 84 % gesättigten aliphatischen und 16 % naphthenischen Kohlenwasserstoffen mit einem Siedebereich von 192 bis 254 °C), 39,45 g eines Emulgators (Umsetzungsprodukt aus 1 Mol Oleylglycidylether, 1 Mol Glycerin und 2 Mol Ethylenoxid, hergestellt nach DE-OS 25 57 324) zugibt und anschließend 213,5 g des gemäß Beispiel 3 erhaltenen gereinigten N-Vinylformamids zufügt. Dann wird 1 Stunde lang Stickstoff bei Raumtemperatur unter Rühren durch die Mischung geleitet. Sie wird dann unter Rühren bei einer Rührerdrehzahl von 300 Umdrehungen pro Minute auf 45 °C erhitzt und mit 0,106 g 2,2′-Azobis(2,4-dimethylvaleronitril) gelöst in 0,226 g Aceton versetzt. Das Reaktionsgemisch wird dann unter kontinuierlichem Rühren auf 60 °C erwärmt und in dem Temperaturbereich von 60 bis 65 °C polymerisiert. Die Polymerisationsdauer beträgt etwa 2 Stunden. Danach fügt man nochmals 0,106 g 2,2′-Azobis(2,4-dimethylvaleronitril) in 0,226 g Aceton gelöst hinzu und hält das Reaktionsgemisch noch 2 Stunden zur Nachpolymerisation auf 60 °C. Der Feststoffgehalt der Polymeremulsion wird durch eine Fällung des Polymeren in Aceton bestimmt. Er beträgt 20,4 %. Der K-Wert des Polymeren beträgt 248.

Vergleichsbeispiel 1

Der in Beispiel 1 angegebenen Kolonne werden pro Stunde 8,0 kg einer Mischung aus 95 % N-Vinylformamid, 2,5 der Verbindung der Formel I und 2,5 % höhersiedender Bestandteil zudosiert. Am Kopf der Kolonne wird ein Druck von 10 mbar aufrechterhalten. Mit der Umwälzpumpe im Sumpfkreislauf werden stündlich 400 l umgepumpt. Der Fallfilmverdampfer muß dabei auf einer Temperatur von 170 °C gehalten werden.

Am Kopf der Kolonne wird ein Rücklaufverhältnis von 3 : 1 eingestellt. Unter diesen Bedingungen können stündlich 6,35 kg reines N-Vinylformamid abdestilliert werden. Im Sumpfbereich stellt sich unter stationären Bedingungen eine Verweilzeit von 1,9 Stunden bei einem Druck von 19 mbar und eine Temperatur von 140 °C ein. Aus dem Sumpf werden stündlich 1,65 kg Sumpfprodukt abgezogen, das neben den ursprünglichen je 0,2 kg der Verbindung der Formel I und hochsiedenden Bestandteilen 1,15 kg Oligomere und Polymere des N-Vinylformamids und 0,1 kg monomeres N-Vinylformamid entsprechend einem Gehalt von 6 % enthält. Damit gehen 15,1 % des ursprünglich vorhandenen N-Vinylformamids durch Polymerisation verloren. Zusätzlich wird noch 1,3 % N-Vinylformamid der ursprünglich vorhandenen Menge mit dem Sumpf als Monomer abgezogen. Nach 28stündigem Betrieb nimmt der Druckverlust in der Kolonne durch beginnende Popcornpolymerisation zu. Nach weiteren 3 Stunden ist die Kolonne zupolymerisiert.

Vergleichsbeispiel 2

Analog dem Vergleichsbeispiel 1 werden der

Kolonne 8,0 kg einer Mischung aus 92 % N-Vinylformaid, 3 % der Verbindung der Formel II und 5 % Hochsieder zugeführt. Stündlich werden am Kopf der Kolonne 6,22 kg reines N-Vinylformamid abdestilliert. Im Sumpfbereich stellt sich unter stationären Bedingungen eine Verweilzeit von 1,7 Stunden und eine Temperatur von 140 °C ein. Es werden stündlich 1,78 kg Sumpfprodukt ausgetragen, das neben dem ursprünglichen Gehalt an Verbindung der Formel II und Hochsiedern 1,1 kg Oligomere und Polymere und außerdem 2 Gew.-% monomeres N-Vinylformamid enthält. Damit gehen insgesamt 15,4 % des zugefahrenen N-Vinylformamids verloren.

Vergleichsbeispiel 3

Adiabatischer Druckwärmestautest mit N-Vinylformamid

80 g reines N-Vinylformamid wurden in einem mit einer Berstscheibe ausgestatteten Autoklaven auf eine Temperatur von 100 °C erhitzt. Dabei stellte sich ein Überdruck von 1 bar ein. Zur Verringerung von Wärmeverlusten wurde bei der nach ca. 1 Std. beginnenden exothermen Reaktion die Ofentemperatur der Produkttemperatur nachgeführt. Nach einer Lagerung von 1,5 Stunden erreichte die Produkttemperatur 136 °C, nach weiteren 10 Minuten 282 °C, wobei der Druck auf über 50 bar anstieg und die Berstscheibe ausgelöst wurde.

Der Druckwärmestautest zeigt, daß N-Vinylformamid thermisch sehr empfindlich ist und beim Erhitzen die Gefahr einer explosionsartigen Polymerisation besteht.

**Patentansprüche**

**Patentansprüche für folgende Vertragsstaaten : BE, CH, DE, FR, GB, GR, IT, LI, LU, NL, SE**

1. Verfahren zur Reinigung von N-Vinylformamid durch fraktionierte Destillation des N-Vinylformamids in einer Kolonne unter vermindertem Druck, dadurch gekennzeichnet, daß man die Destillation in Gegenwart von Formamid unter einem Druck von 0,5 bis 30 mbar, gemessen am Kopf der Kolonne, durchführt und die Destillation so steuert, daß als Destillat N-Vinylformamid mit einem Gehalt von 0,1 bis 15 Gew.-% Formamid anfällt.

2. Verfahren nach Anspruch 1, dadurch gekennzeichnet, daß man die Destillation kontinuierlich unter einem Druck von 0,5 bis 30 mbar, gemessen am Kopf der Kolonne, so durchführt, daß als Destillat N-Vinylformamid mit einem Gehalt von 1 bis 6 Gew.-% Formamid anfällt.

3. Verfahren nach den Ansprüchen 1 und 2, dadurch gekennzeichnet, daß man die Destillation, bezogen auf das dabei eingesetzte N-Vinylformamid, in Gegenwart von 1 bis 70 Gew.-% Formamid durchführt.

4. Hompolymerisate von N-Vinylformamid mit einem K-Wert von oberhalb 200 (bestimmt nach Fikentscher in 5 %iger wäßriger Kochsalzlösung bei 25 °C und einer Polymerkonzentration von 0,1 Gew.-%), dadurch gekennzeichnet, daß sie erhältlich sind durch Polymerisation von N-Vinylformamid, das gemäß Anspruch 1 gereinigt ist.

**Patentansprüche für folgende Vertragsstaaten : AT, ES**

1. Verfahren zur Reinigung von N-Vinylformamid durch fraktionierte Destillation des N-Vinylformamids in einer Kolonne unter vermindertem Druck, dadurch gekennzeichnet, daß man die Destillation in Gegenwart von Formamid unter einem Druck von 0,5 bis 30 mbar, gemessen am Kopf der Kolonne, durchführt und die Destillation so steuert, daß als Destillat N-Vinylformamid mit einem Gehalt von 0,1 bis 15 Gew.-% Formamid anfällt.

2. Verfahren nach Anspruch 1, dadurch gekennzeichnet, daß man die Destillation kontinuierlich unter einem Druck von 0,5 bis 30 mbar, gemessen am Kopf der Kolonne, so durchführt, daß als Destillat N-Vinylformamid mit einem Gehalt von 1 bis 6 Gew.-% Formamid anfällt.

3. Verfahren nach den Ansprüchen 1 und 2, dadurch gekennzeichnet, daß man die Destillation, bezogen auf das dabei eingesetzte N-Vinylformamid, in Gegenwart von 1 bis 70 Gew.-% Formamid durchführt.

4. Verfahren zur Herstellung von Homopolymerisaten von N-Vinylformamid mit einem K-Wert von oberhalb 200 (bestimmt nach Fikentscher in 5%iger wäßriger Kochsalzlösung bei 25 °C und einer Polymerkonzentration von 0,1 Gew.-%), dadurch gekennzeichnet, daß man das nach Anspruch 1 gereinigte N-Vinylformamid, das 0,1 bis 15 Gew.% Formamid enthält, polymerisiert.

**Claims**

**Claims for the following Contracting States : BE, CH, DE, FR, GB, GR, IT, LI, LU, NL, SE**

1. A process for purifying N-vinylformamide by fractional distillation of N-vinylformamide in a column under reduced pressure, wherein the distillation is carried out in the presence of formamide under from 0.5 to 30 mbar, measured at the top of the column, and the distillation is controlled so that N-vinylformamide containing from 0.1 to 15% by weight of formamide is obtained as the distillate.

2. A process as claimed in claim 1, wherein the distillation is carried out continuously under from 0.5 to 30 mbar, measured at the top of the column, so that N-vinylformamide containing from 1 to 6% by weight of formamide is obtained as the distillate.

3. A process as claimed in claim 1 or 2, wherein the distillation is carried out in the presence of from 1 to 70% by weight, based on the N-vinylformamide used, of formamide.

4. A homopolymer of N-vinylformamide having a K value greater than 200 (determined by the Fikentscher method in 5% aqueous sodium chloride solution at 25°C and at a polymer concentration of 0.1% by weight), which is obtainable by polymerization of N-vinylformamide purified according to claim 1.

**Claims for the following Contracting States : AT, ES**

1. A process for purifying N-vinylformamide by fractional distillation of N-vinylformamide in a column under reduced pressure, wherein the distillation is carried out in the presence of formamide under from 0.5 to 30 mbar, measured at the top of the column, and the distillation is controlled so that N-vinylformamide containing from 0.1 to 15% by weight of formamide is obtained as the distillate.

2. A process as claimed in claim 1, wherein the distillation is carried out continuously under from 0.5 to 30 mbar, measured at the top of the column, so that N-vinylformamide containing from 1 to 6% by weight of formamide is obtained as the distillate.

3. A process as claimed in claim 1 or 2, wherein the distillation is carried out in the presence of from 1 to 70% by weight, based on the N-vinylformamide used, of formamide.

4. A process for preparing a homopolymer of N-vinylformamide having a K value greater than 200 (determined by the Fikentscher method in 5% aqueous sodium chloride solution at 25°C and at a polymer concentration of 0.1% by weight), wherein the N-vinylformamide purified according to claim 1 and containing from 0.1 to 15% by weight of formamide is polymerized.

**Revendications**

**Revendications pour les Etats contractants suivants : BE, CH, DE, FR, GB, GR, IT, LI, LU, NL, SE**

1. Procédé de purification du N-vinylformamide par distillation fractionnée du N-vinylformamide dans une colonne, sous pression réduite, caractérisé en ce que l'on entreprend la distillation en présence de formamide, sous une pression de 0,5 à 30 mbars, mesurée en tête de la colonne, et en ce que l'on conduit la distillation de manière à ce que le distillat obtenu soit du N-vinylformamide d'une teneur en formamide de 0,1 à 15% en poids.

2. Procédé suivant la revendication 1, caractérisé en ce que l'on entreprend la distillation en continu, sous une pression de 0,5 a 30 mbars, mesurée en tête de la colonne, de façon à ce que le distillat obtenu soit du N-Vinylformamide d'une teneu en formamide de 1 à 6% en poids.

3. Procédé suivant les revendications 1 et 2, caractérisé en ce que l'on entreprend la distillation en présence de 1 a 70% en poids de formamide, par rapport au N-vinylformamide mis en oeuvre.

4. Homopolymères du N-vinylformamide, d'une valeur K supérieure à 200 (comme déterminée selon Fikentscher en solution aqueuse à 5% de sel de cuisine, à 25°C, et à une concentration en polymère de 0,1% en poids), caractérisés en ce qu'on peut les obtenir par la polymérisation du N-vinylformamide, purifié selon la revendication 1.

**Revendications pour les Etats contractants suivants : AT, ES**

1. Procédé de purification du N-vinylformamide par distillation fractionnée du N-vinylformamide dans une colonne, sous pression réduite, caractérisé en ce que l'on entreprend la distilation en présence de formamide, sous une pression de 0,5 à 30 mbars, mesurée en tête de la colonne, et en ce que l'on conduit la distillation de manière a ce que le distillat obtenu soit du N-vinylformamide d'une teneur en formamide de 0,1 à 15% en poids.

2. Procédé suivant la revendication 1, caractérisé en ce que l'on entreprend la distillation en continu, sous une pression de 0,5 à 30 mbars, mesurée en tête de la colonne, de façon à ce que le distillat obtenu soit du N-vinylformamide d'une teneur en formamide de 1 à 6% en poids.

3. Procédé suivant les revendications 1 et 2, caractérisé en ce que l'on entreprend la distillation en présence de 1 à 70% en poids de formamide, par rapport au N-vinylformamide mis en oeuvre.

4. Procédé de préparation d'homopolymères du N-vinylformamide, d'une valeur K supérieure à 200 (comme déterminée selon Fikentscher en solution aqueuse à 5% de sel de cuisine, à 25 °C, et à une concentration en polymère de 0,1% en poids), caractéricés en ce que l'on procède à la polymérisation du N-vinylformamide obtenu selon la revendication 1, qui contient de 0,1 à 15% en poids de formamide.